# EUROPEAN PATENT SPECIFICATION

(11) **EP 3 914 151 B1**
(45) Date of publication and mention of the grant of the patent: **01.04.2026**
(21) Application number: 20744877.0
(22) Date of filing: 21.01.2020
(51) Int. Cl.: A61B 5/03, A61B 17/34, A61M 25/01, A61M 25/06, A61M 25/09, A61M 25/14, A61B 1/00, A61B 1/01, A61M 25/00, A61B 17/00

(54) **MICROCATHETER FOR THERAPEUTIC AND/OR DIAGNOSTIC INTERVENTIONS IN THE SUBARACHNOID SPACE**
MIKROKATHETER FÜR THERAPEUTISCHE UND/ODER DIAGNOSTISCHE EINGRIFFE IM SUBARACHNOIDRAUM
MICROCATHÉTER POUR INTERVENTIONS THÉRAPEUTIQUES ET/OU DE DIAGNOSTIC DANS L'ESPACE SOUS-ARACHNOÏDIEN

(30) Priority: 22.01.2019 US 201962795350 P
(43) Date of publication of application: 01.12.2021
(73) Proprietor: The Trustees of The University of Pennsylvania, Philadelphia, PA 19104 (US)
(72) Inventor: PUKENAS, Bryan, Haddonfield, NJ 08033 (US)
(74) Representative: Robba, Pierpaolo
(86) International application number: PCT/US2020/014402
(87) International publication number: WO 2020/154287

(56) References cited:
- WO-A1-2015/011986
- WO-A1-2016/168605
- WO-A1-2018/160966
- US-A- 5 396 880
- US-A- 6 146 355
- US-A1- 2003 097 082
- US-A1- 2016 367 120
- US-A1- 2019 000 305

## Description

### BACKGROUND

The conventional standard of care for therapeutic and diagnostic interventions for a diseased brain and/or spinal cord of a patient is open surgery.

For example, about 3-5 million people in the United States alone have brain aneurysms, and about 30-40 percent of aneurysms are treated with open surgery. In addition, about 80,000 new brain tumors are diagnosed each year, with surgery and systemic chemotherapy/radiation being the typical treatment.

There has been hesitation to use catheters in the spinal canal subarachnoid space of a patient having a diseased brain and/or spinal cord because of potential injury to the spinal cord.

US 5,396,880 A discloses a system for direct visualization of the spine and the epidural and/or intra-discal space to facilitate diagnosis and treatment of spinal conditions and that is adapted for percutaneous introduction into the spinal space. The system includes a disposable flexible catheter, a fiber-optic bundle disposed within the catheter which is connected to a light source and camera. The bundle is removably and adjustably connected to the proximal end of the catheter to permit rotation of the bundle relative to the catheter. A mechanism for controllably deflecting the tip of the catheter is provided to vary the viewing angle of the fiber-optic bundle within and to assist in steering the catheter through the spinal space. The mechanism includes a deflection wire extending through the catheter and affixed at the distal end thereof. The proximal end of the deflection wire is affixed to a sleeve which is slidably disposed around the catheter and within a housing. The housing includes an internal flange that defines a stop surface which is contacted by the sleeve as the catheter and deflection wire is moved in a first direction. After the sleeve contacts the stop surface, further movement of the catheter in the first direction causes tension in the wire between the sleeve and the wire's securement to the catheter, thereby bending the catheter tip in the direction of the securement. The catheter can be rotated with the tip in its deflected position to provide a conical viewing region within the spinal space.

US 2016/367120 A1 discloses a device for performing surgical procedures, such a intracardiac procedures or neurosurgical procedures, includes a solid optical window formed of a transparent, compliant material, wherein the solid optical window includes a proximal side and a distal side, wherein a distal face of the solid optical window is configured to approach tissue during a surgical procedure; an imaging system embedded into the solid optical window and positioned to obtain an image through at least a portion of the distal face of the solid optical window; and a tool channel formed through the solid optical window from the proximal side to the distal side of the solid optical window, wherein the tool channel is configured to receive a tool for performing the surgical procedure.

### SUMMARY OF THE INVENTION

The invention is defined by independent claim 1. Various preferred embodiments of the invention can be found in the dependent claims.

A minimally-invasive access and delivery medical device is provided for therapeutic and/or diagnostic interventions within the subarachnoid space of a patient. The medical device includes an elongate, flexible microcatheter having proximal and distal ends, a guidewire extending through the microcatheter and being slidable relative to the microcatheter such that the guidewire is freely extendable from the distal end of the microcatheter to a position beyond the distal end and retractable within the microcatheter and such that the microcatheter is advanceable along the guidewire when the guidewire is extended beyond the distal end of the microcatheter, and a fiber-optic probe extending through the microcatheter from the distal to the proximal end of the microcatheter. The fiber-optic probe has a tip extending adjacent the distal end of the microcatheter and being adjustable relative to the distal end of the microcatheter such that the tip provides an end viewing face directed and/or adjustably directed in a direction toward the guidewire. A camera is connected to the fiber-optic probe adjacent the proximal end of the microcatheter to provide real-time visualization of the contents of the subarachnoid space of the patient and guidewire during navigation of the distal end of the microcatheter via the guidewire to the brain of the patient.

According to another aspect, the medical device may also include a steering wire extending through the microcatheter from the proximal to the distal end of the microcatheter. The steering wire may be fixed to the distal end to enable steering of the distal end of the microcatheter.

Other aspects of the invention will be readily apparent from the following detailed description.

### BRIEF DESCRIPTION OF THE DRAWINGS

FIG. 1 is a perspective view of the distal end of a microcatheter (with multi-lumen protective tubing removed) according to an embodiment.
FIG. 2 is a perspective view of the distal end of a microcatheter of FIG. 1 according to an embodiment.
FIG. 3 is a cross-section view of the multi-lumen protective tubing of FIG. 2 perpendicular to the longitudinal axis of the tubing according to an embodiment.
FIG. 4 is a perspective view of the distal end of a microcatheter of FIG. 2 according to an embodiment.
FIG. 5 is a perspective view of a proximal end of a microcatheter interconnected to a camera caddy according to an embodiment.
FIG. 6 is a perspective view of a proximal end of a microcatheter with the camera caddy removed according to an embodiment.
FIG. 7 is an exploded perspective view of the proximal end of the microcatheter according to an embodiment.
FIG. 8 is an enlarged perspective view of an internal part of the proximal end of the microcatheter according to an embodiment.

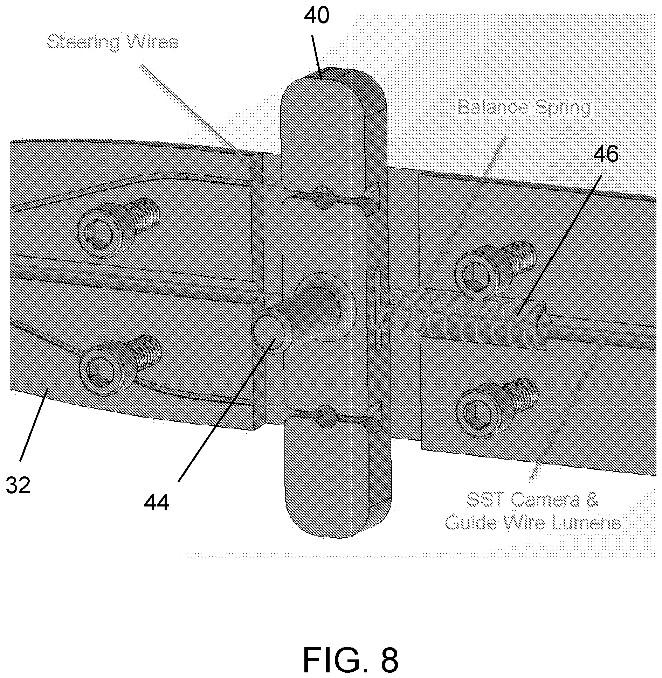

FIG. 8 is an enlarged perspective view of an internal part of the proximal end of the microcatheter according to an embodiment.

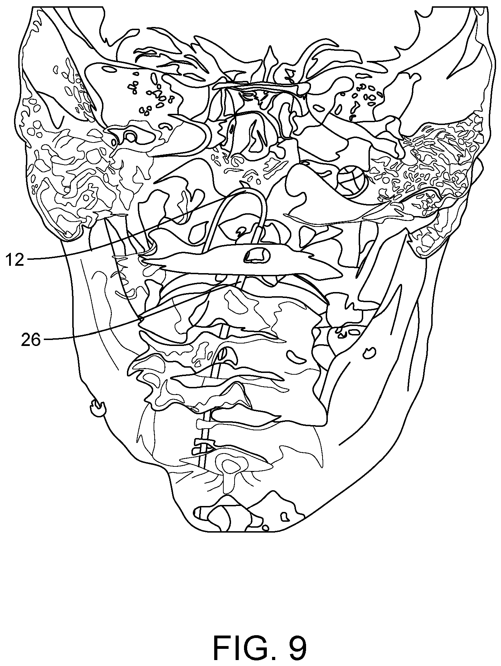

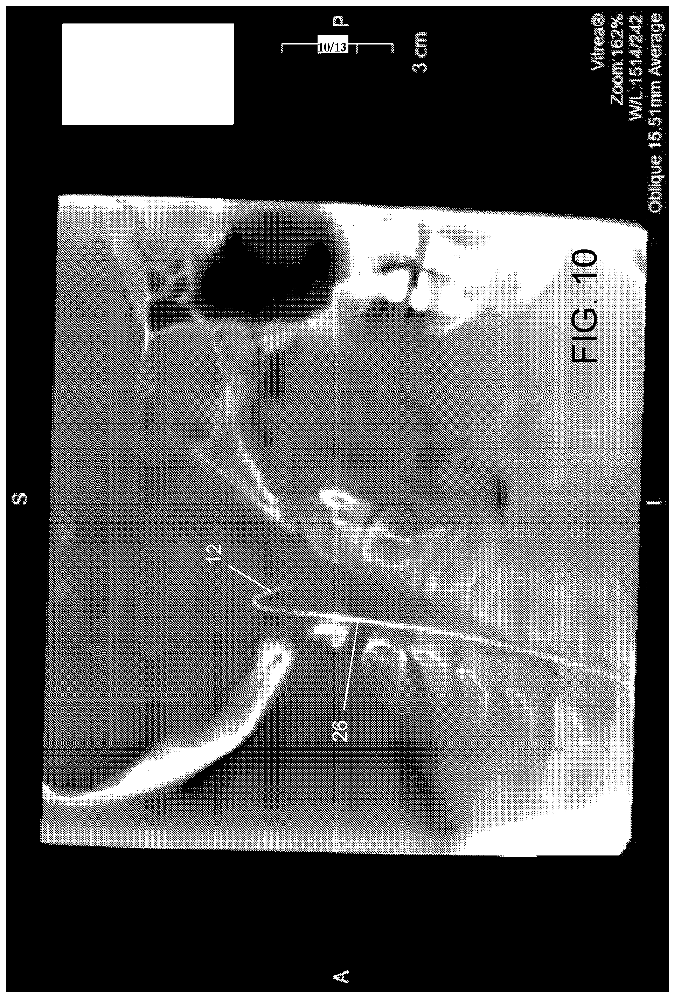

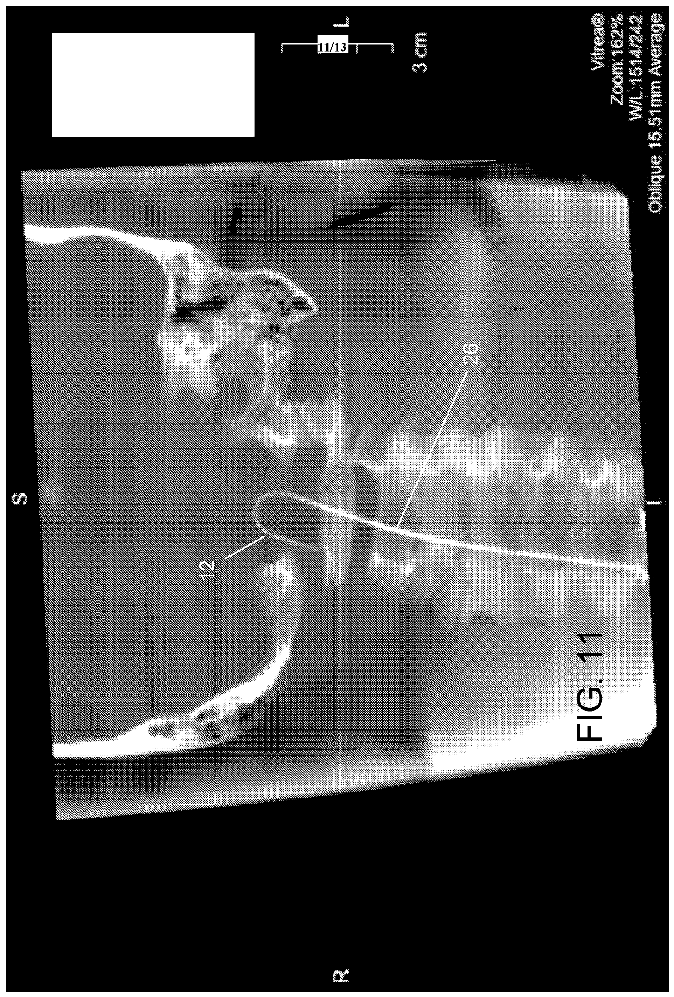

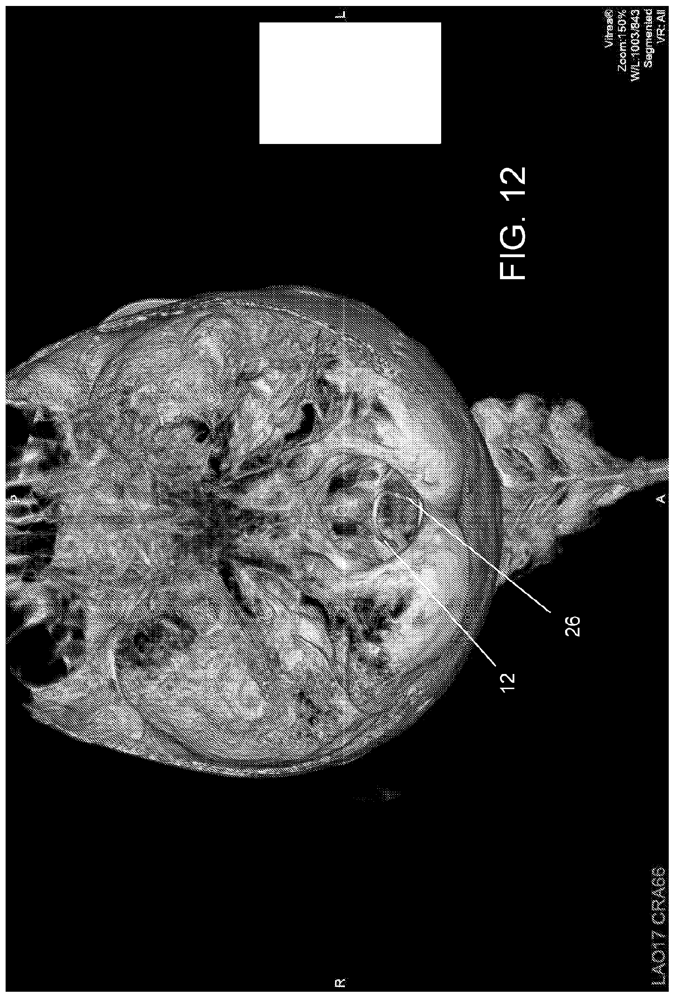

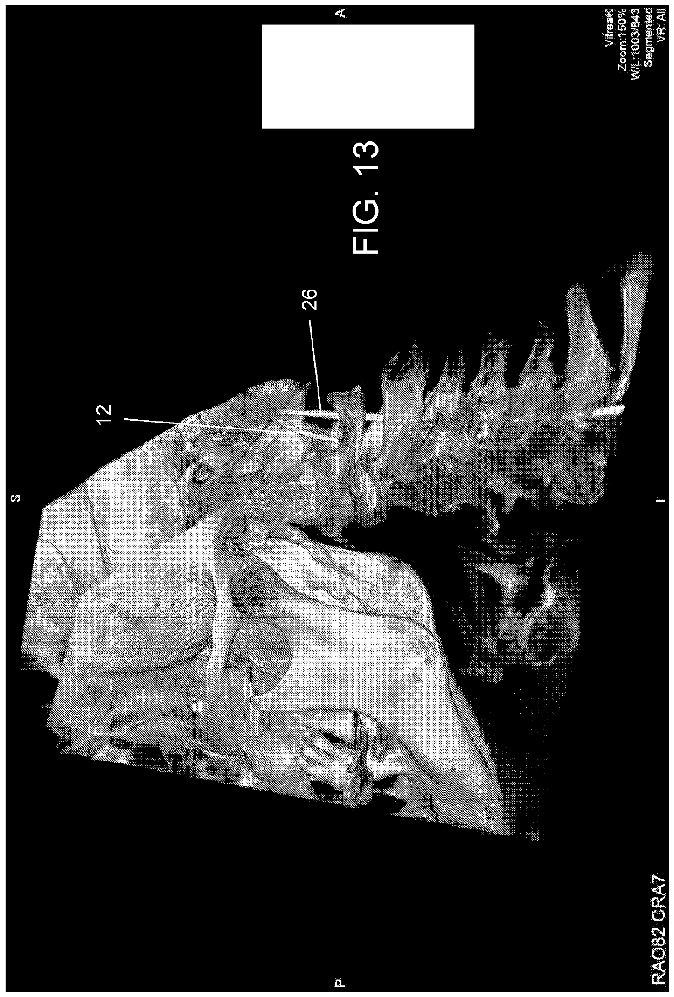

FIGs. 9-13 are radiographic images of the microcatheter in use extending within the subarachnoid space.

### DETAILED DESCRIPTION OF THE INVENTION

### Definitions

The following definitions are provided. It is to be noted that the term "a" or "an" refers to one or more. As such, the terms "a" (or "an"), "one or more," and "at least one" are used interchangeably herein.

The words "comprise", "comprises", and "comprising" are to be interpreted inclusively rather than exclusively. The words "consist", "consisting", and its variants, are to be interpreted exclusively, rather than inclusively. While various embodiments in the specification are presented using "comprising" language, under other circumstances, a related embodiment is also intended to be interpreted and described using "consisting of" or "consisting essentially of" language.

As used herein, the term "about" means a variability of 10% from the reference given, unless otherwise specified.

A "patient" is a mammal, e.g., a human, mouse, rat, guinea pig, dog, cat, horse, cow, pig, or non-human primate, such as a monkey, chimpanzee, baboon or gorilla. In one embodiment, the patient is a human.

The "subarachnoid space" is the space between the arachnoid membrane and pia mater that covers the central nervous system, surrounds the brain and spinal cord, and contains cerebrospinal fluid.

The term "real-time visualization" refers to viewing images within milliseconds of the images actually being taken so that it is available virtually immediately as feedback.

Unless otherwise specified, the term "central nervous system" includes the brain, the spinal cord, and the spinal nerves and their components within the subarachnoid space (spinal ganglia). In certain embodiments, the brain will be the specific target of treatment and the other parts of the central nervous system will not be targeted for therapy.

As used herein, the term "peripheral nervous system" refers to nerves and ganglia outside the brain and spinal cord.

Unless defined otherwise in this specification, technical and scientific terms used herein have the same meaning as commonly understood by one of ordinary skill in the art and by reference to published texts, which provide one skilled in the art with a general guide to many of the terms used in the present application.

### Description of Embodiments of a Microcatheter

Embodiments of a medical device are disclosed herein which enable therapeutic and diagnostic interventions in the subarachnoid space of a patient in a minimally-invasive manner. The medical device is provided in the form of a microcatheter which may be inserted into the subarachnoid space by a small puncture, such as a lumbar puncture in the lower back of the patient, and then navigated within the subarachnoid space through cerebrospinal fluid, such as along the spinal canal toward the cisterna magna at the base of the patient's brain or any other location within the subarachnoid space. The microcatheter includes a guidewire, optionally a steering wire or wires, optionally a fiber-optic lighting element, and a fiber-optic probe or, in an example outside the scope of the invention, other micro-camera, that enables the contents of the subarachnoid space within cerebrospinal fluid (CSF), which is typically clear, to be viewed in real-time as the microcatheter is navigated within the subarachnoid space.

Embodiments of the above referenced minimally-invasive access and therapy delivery system reduce morbidities and cost associated with open surgery. In addition, this approach allows for patients to be treated in fluoroscopy suites (outside of an operating room) thereby greatly lowering the cost of such procedures. Patients should also have faster recovery times, since the access site may be in the lumbar spine and only a bandage or suture would be needed for wound care, entirely eliminating the need to recover from a craniotomy.

By way of example, and not be way of limitation, the above referenced minimally- invasive access and therapy delivery system could be used for delivery of gene therapy, drug delivery (e.g. direct treatment of brain for epilepsy, brain tumors, etc.), probes, laser ablation, medical tools such as a lasso or electrode, and the like to a diseased brain. In addition, the system could be used for other applications, such as: electrical ablation probe; electrical probe; Ph sensor; gastrointestinal (GI) imaging (replaces capsule); lasso for aneurysm; thermal ablation; cryotherapy; scar tissue removal (fertility); bladder access and imaging; lung access and imaging; radiation emitting probe; lymphatic imaging and access; egg harvesting; and the like.

According to one embodiment, the above referenced minimally-invasive access and therapy delivery system is used to access the subarachnoid space for delivering novel gene therapies to treat a variety of congenital and other diseases. Novel gene therapies are being developed to treat a variety of diseases, and circumventing the blood brain barrier is essential for these therapies to be effective (i.e., central nervous system gene therapy vectors may need to be placed directly into the cisterna magna (at the skull base) for greater transmission). Such a delivery system, which bypasses the blood brain barrier, may reduce the amount (e.g., volume and/or dose) of costly gene therapy required. In certain embodiments, the devices provided herein may improve transduction 10-fold to 100-fold compared to regular lumbar delivery of gene therapy.

According to other embodiments, the system may be used in a procedure to fix an aneurysm. For instance, the system may be used to place electrodes directly on the brain to find an epileptic focus or may be used to have brain tumors treated with laser ablation. In addition, the system may be used for direct infusion of chemotherapy and catheter directed tumor ablation. Of course, these are only a few examples of the benefits of a dedicated intrathecal treatment system according to embodiments disclosed herein.

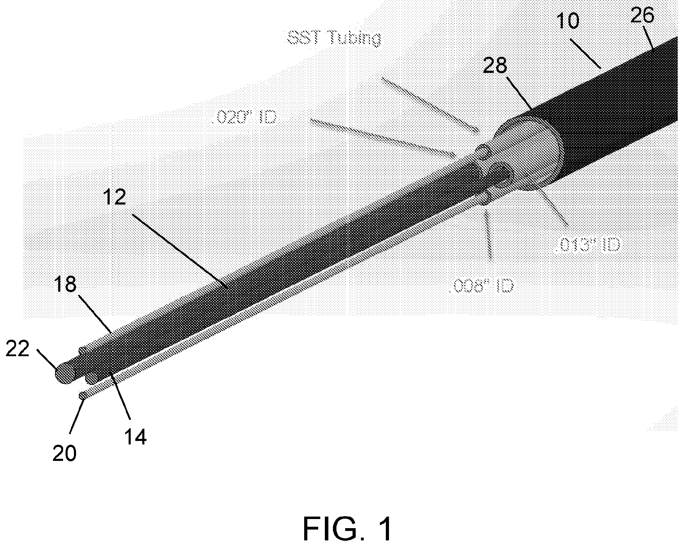

According to one embodiment, a microcatheter 10 is provided having a distal end 12 as shown in FIGs. 1-4. A guidewire 14 extends through the microcatheter 10 from a proximal end 16 to and through the distal end 12 and may be extended or retracted relative to the microcatheter thereby permitting the microcatheter 10 to be advanced along the guidewire. Thus, in use, the guidewire 14 may be inserted into patient, such as via lumbar puncture, and then the microcatheter 10 may be advanced along the guidewire.

One or more steering wires, 18 and 20, may extend from the proximal end 16 to the distal end 12 within the microcatheter 10. In the embodiment shown in FIGs. 1-4, there are two steering wires 18 and 20 (which may be formed by opposite ends of the same wire or may be completely separate wires). Of course, the microcatheter may be provided with a single steering wire, with more than two steering wires, or with no steering wire. Thus, according to an embodiment, the microcatheter may be provided without a steering wire.

The steering wire or wires, 18 and 20, may be secured to the distal end 12 and a force applied to the steering wires at the proximal end 16 of the microcatheter 10 may be used to direct the distal end 12 in one direction or the other to enable safe navigation within the subarachnoid space. For instance, navigation may take place within the spinal canal of a patient, such as from a lumbar puncture to the cistema magna. Of course, the microcatheter may be navigated to other locations within the subarachnoid space or within other spaces. In addition, the steering wire may permit the distal end 12 to form a relatively tight bend or curve, for instance of about 180° as best shown in the radiographic images of FIGs. 9-13. In one contemplated embodiment, one of the steering wires may be replaced with a fiber optic light transmission element or the like (not shown) for permitting light to be projected from the distal end of the microcatheter.

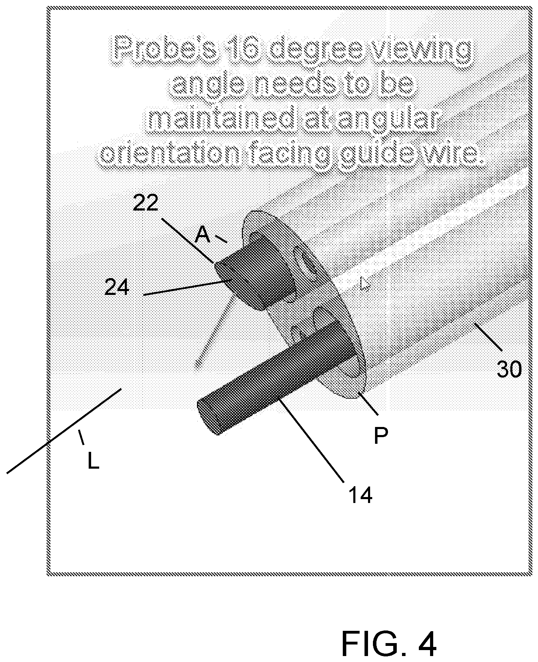

A fiber-optic or like probe 22 also extends from the proximal end 16 to the distal end 12 within the microcatheter 10. As best shown in FIG. 4, the tip of the fiber-optic probe 22 has a a viewing face 24 which is directed or directable toward the guidewire 14, such as at an angle. At least in the distal end 12, the fiber-optic probe 22 may be secured such that viewing face 24 always faces the guidewire 14 so that the guidewire 14 is always visualized when navigating the microcatheter within the subarachnoid space. The positioning of the fiber-optic probe 22 is adjustable, such as be being rotated about a longitudinal access of the probe within the lumen of the microcatheter and relative to the microcatheter or by other means.

According to the embodiment illustrated in FIG. 4, the angle "A" of the viewing face 24 relative to a plane "P" extending perpendicular through a longitudinal axis "L" of the distal end 12 may be within a range of 0° to 90°, more preferably within a range of 12° to 20°, or about 16°. Thus, when the viewing face 24 is directed toward the guidewire, the viewing face 24 has an angular orientation relative to the guidewire and thus is able to capture the guidewire in an image produced. Of course, other means for placing the guidewire in the image capturing area of the fiber-optic probe may be utilized.

As best shown in FIG. 1, the microcatheter 10 may have an elongate intermediate section 26 having an outer protective tubing 28 in which the guidewire 14, steering wires 18 and 20, and fiber-optic probe 22 extend to the proximal end 16 of the microcatheter 10. The tubing 28 may have a single lumen in which the guidewire 14, steering wires 18 and 20, and the fiber-optic probe 22 extend. Each of the guidewire 14, steering wires 18 and 20, and fiber-optic probe 22 may extend within separate smaller diameter tubing within the outer protective tubing 26. See FIG. 1. Alternatively, the tubing 28 may be in the form of an extruded multi-lumen tube.

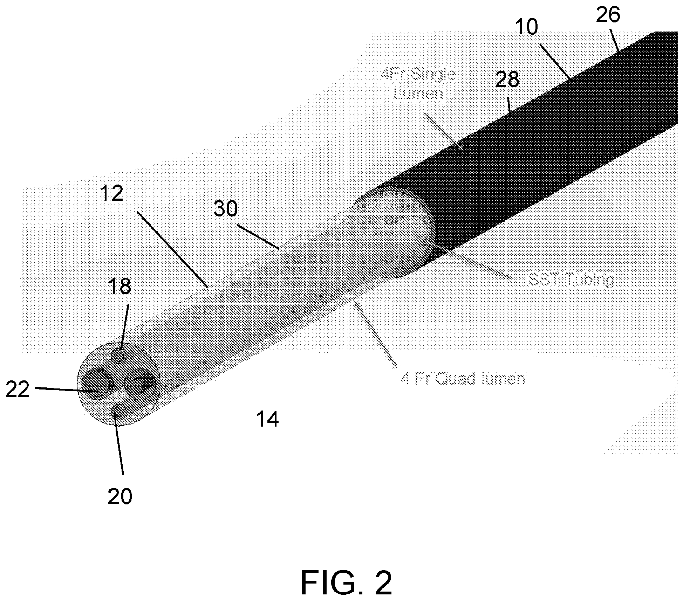

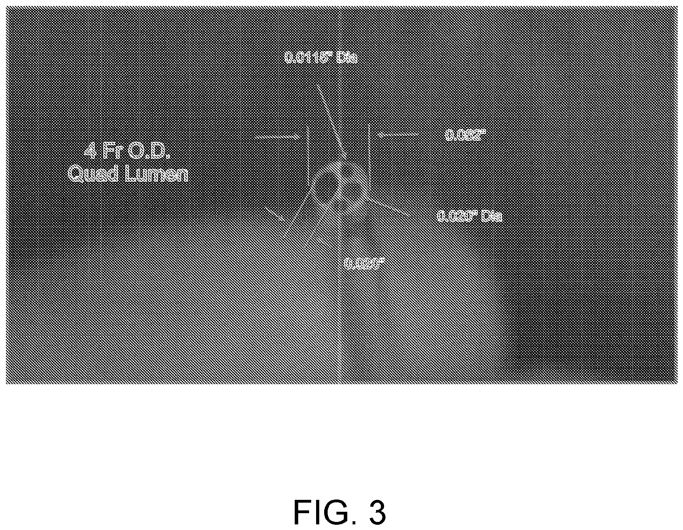

A multi-lumen protective tubing 30 forming the distal end 14 may extend from the tubing 28. As best shown in FIGs. 2 and 3, the tubing 30 forming the distal end 14 has four separate lumens, one each for the guidewire 14, steering wires 18 and 20, and fiber-optic probe 22. The steering wires, 18 and 20, may be fixed relative to the tubing 30 so that the flexible distal end 12 of the microcatheter is steerable. In contrast, the guidewire 14 is permitted to slide relative to the tubing 28 so that it can be extended and retracted relative to the tubing 30.

According to an embodiment, the tubing 30 that forms the distal end may be more or less easily flexed by the steering wires than the tubing 28. Thus, the microcatheter may have varying degrees of flexibility/ stiffness throughout its length. For instance, as best shown in FIGs. 9-13, the distal end 14/tubing 30 may be flexed with use of the steering wires to the extent of forming a 180° curve or the like while the tubing 28 remains in a relatively stable and straight condition. Thus, tubing 30 of the distal end 12 may be made of material more flexible than the tubing 28, and forces exerted by the steering wires, 18 and 20, have a greater impact on adjusting the shape and direction of tubing 30 than on the more rigid tubing 28. Alternatively, tubing 30 may be stiffer than the tubing 28 or may be of the same stiffness and flexibility.

By way of example, and not by way of limitation, the tubing 28 and 30 may be of a size of less than 5 French (Fr), 3.5 Fr or less, or 3 Fr or less. These sizes correspond to outer diameters of less than 1.66 mm (0.07 inch), 1.167 mm (0.046 inch) or less, and 1 mm (0.039 inch) or less. For instance, as shown in FIG. 3, the size of the microcatheter is 4 Fr (i.e., 1.33 mm (0.052 inch) outer diameter), the lumen for the guidewire may have a diameter of 0.508 mm (0.020 inch), each lumen for each of the steering wires may have a diameter of 0.292 mm (0.0115 inch), and the lumen for the fiber-optic probe may be oval and have a smallest diameter of about 0.508 mm (0.020 inch).

Of course, these are provided as examples and other sizes and shapes may be used.

Further, as shown in FIG. 3, the lumens for the steering wires are on opposite sides of the tubing 30 so that the direction of the distal end 10 may be pulled in one of these opposite directions, and the lumens for the guidewire and fiber-optic probe are on opposite sides of the tubing 30 so that the viewing face 24 of the probe 22 may be directed at and have a clear view of the guidewire 14 in any image provided by the probe 22. In addition, the combined length of the tubing 28 and 30 may be at least about 1.5 meters (59 inches) or longer; however, shorter lengths may also be utilized depending upon desired end use.

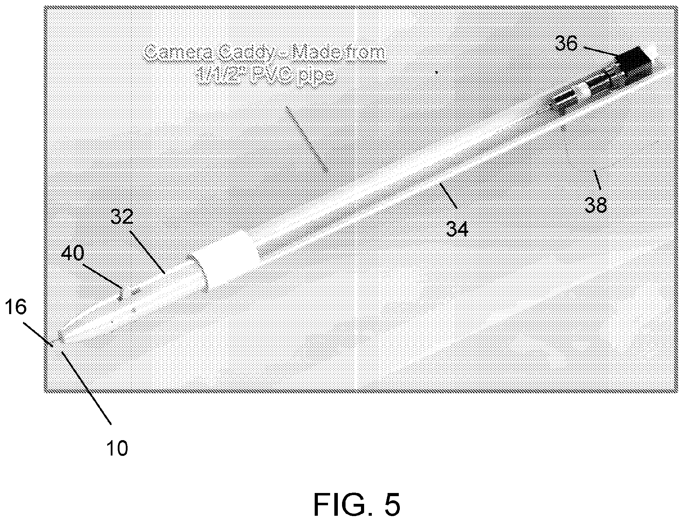

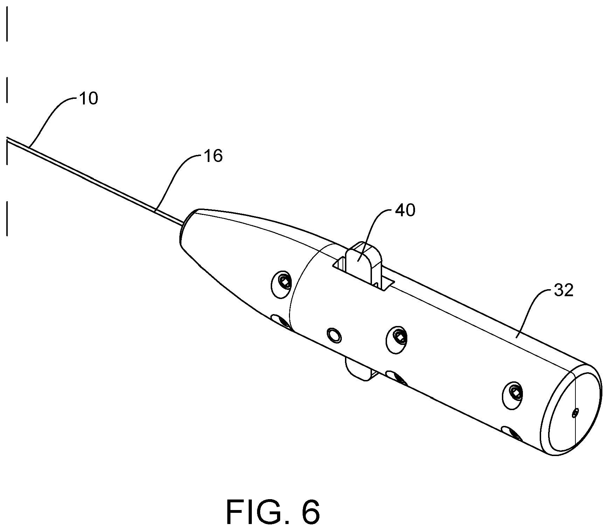

The proximal end 16 of the microcatheter 10 is shown in FIGs. 5-8. The tubing 28 extends into one end of a handle body 32 and a caddy 34 for housing a camera 36 extends from an opposite end of the handle body 32. The fiber-optic probe 22 extends through the handle body 32 and camera caddy 34 and connects to a camera 36. Images obtained via use of the probe 22 and camera 36 may be communicated to a computer, display screen, or the like via a wire or cable 38 extending externally of the camera caddy 34 or via a wireless transmitter (not shown). Thus, images, including still images and video, adjacent the distal end 14 of the microcatheter may be viewed in real-time by an operator of the microcatheter so that the distal end 14 may be navigated, controlled, and positioned, as desired by the operator. For instance, see the microcatheter 10 and distal end 14 thereof extended and positioned as shown in FIGs. 9-13 relative to a spine and brain. [0044] The handle body 32 includes a control lever 40 or the like which may be used to steer the distal end 14 of the microcatheter 10. For instance, by toggling the control lever 40 in a given direction the control lever 40 may cause a pulling force to be applied to a steering wire, 18 or 20, so that the distal end 14 is steered, advanced, and/or curved in a particular direction. Thus, in use, an operator views the images captured at the distal end in real-time and controls the direction of the distal end 14 of the microcatheter 10 to navigate the subarachnoid space.

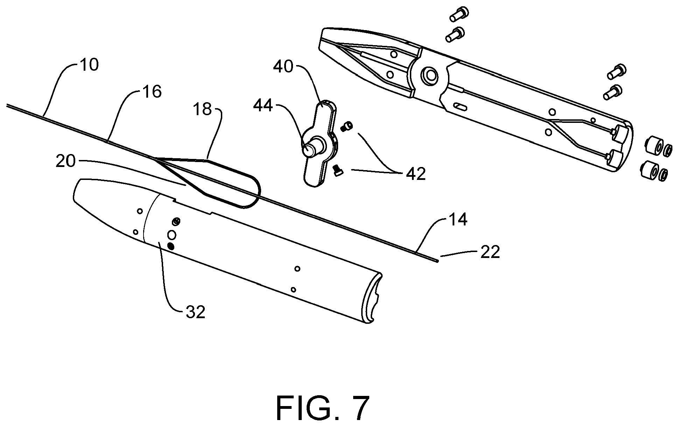

FIGs. 7 and 8 provide an example of an assembly for forming the handle body 32. The guidewire 14 and fiber-optic probe 22 extend the entire length of the handle body 32 and exit into the caddy 34. However, in the embodiment shown in FIGs. 7 and 8, the steering wires, 18 and 20, form opposite ends of the same integral wire and form a loop within the handle body 32 about the control lever 40. The guidewires are secured to opposite ends of the control lever 40 with set screws 42 or the like, and the control lever 40 includes a center shaft 44 which permits the control lever 40 to be rotated in a clockwise or counter-clockwise direction relative to the shaft 44. A spring 46 or like resilient element balances and maintains the control lever 40 in a normal position in which the distal end 14 is placed and extended in a relatively straight condition. Thus, when the operator rotates the control lever 40 in a particular direction, this exerts a force on or pulls on one of the steering wires, 18 and 20, thereby causing the distal end 14 of the microcatheter 10 to deflect or curve in a desired direction to a desired extent.

Although not illustrated, a light source (not shown) may also be housed within the caddy 34 and a fiber-optic cable (not shown) may extend through the handle body 32 to the distal end 14 through with light may be projected.

According to an example which is outside the scope of the present invention, a method of accessing and providing a path of delivery to the subarachnoid space of a patient includes the steps of extending the distal end of the above referenced elongate, flexible, multi-lumen microcatheter through a puncture into the subarachnoid space of the patient and navigating the distal end of the microcatheter within the spinal canal of the patient to other locations within the subarachnoid space of the patient. The method may include a step of delivering at least one of gene therapy, a drug, a probe, a medical tool, laser ablation, an electrode, and a lasso to the distal end of the microcatheter. The method may also include a step of steering the distal end of the microcatheter during the navigating step by toggling a control lever on a handle at the proximal end of the microcatheter. The method may further include the step of projecting light from the distal end of the microcatheter with a fiber-optic light element extending through the microcatheter.

According to another example which is outside the scope of the present invention, a method of accessing and providing a path of delivery to the subarachnoid space of a patient includes the steps of extending the distal end of the above referenced elongate, flexible, microcatheter through a puncture in the cistema magna. For gene therapy, navigating to the cistema magna from lumbar puncture may be adequate; however, for some other therapies, it may be advantageous to navigate from the cisterna magna to the surface of the brain (i.e., epilepsy treatment).

The device provided herein minimizes the risk associated with current devices used for delivery of intrathecal therapies and surgical procedures for the brain or other parts of the central nervous system. In particular, use of the device provided herein avoids the risk associated with prior art devices used for intrathecal, and particularly, intracerebroventricular injection.

The device may be used for delivering a therapeutic or other regimen to the central nervous system of a patient in need thereof. Examples of such therapies include, e.g., oncolytic therapy, gene therapy, antisense therapy, immunotherapy, delivery of small molecule drugs, delivery of anesthesia, pain medication, or chemotherapies. Additionally, the device may be used for treatments of aneurysms and/or vascular diseases of the brain and spine, including, without limitation arteriovenous malformations, fistulas, cavernomas, among other conditions. This device may be useful in treating patients with a variety of indications including, without limitation, primary or metastatic cancers of the brain and/or central nervous system, lysosomal storage diseases, movement disorders including but not limited to primary essential tremor, Parkinson's Disease, Alzheimer's Disease, mucopolysaccharidoses (MPS) which include seven subtypes: MPS I, MPS II, MPS III, MPS IV, MPS VI, MPS VII, and MPS IX; spinal muscular atrophy (SMA), Batten disease (neuronal ceroid lipofuscinoses, or NCLs); transmissible spongiform encephalopathies (e.g., Creutzfeldt- Jacob disease), amyotrophic lateral sclerosis (ALS), multiple sclerosis, Huntington disease, Canavan's disease, traumatic brain injury, spinal cord injury, migraine, lysosomal storage diseases, stroke, and infectious disease affecting the central nervous system. Examples of suitable gene therapy and immunotherapy gene products and antibodies are identified, e.g., in WO 2017/075119.

As used herein, the "subject" or "patient" is a mammal, e.g., a human, mouse, rat, guinea pig, dog, cat, horse, cow, pig, or non-human primate, such as a monkey, chimpanzee, baboon or gorilla.

Optionally, the device is useful for delivery of an active drug(s)which is in a pharmaceutically acceptable suspension or solution (e.g., an aqueous based composition), which optionally contains conventional pharmaceutical ingredients, such as preservatives, or chemical stabilizers.

Suitably, the composition may contain water (e.g., saline), a surfactant, and a physiologically compatible salt or mixture of salts. Suitably, the formulation is adjusted to a physiologically acceptable pH, e.g., in the range of pH 6 to 9, or pH 6.5 to 7.5, pH 7.0 to 7.7, or pH 7.2 to 7.8. As the pH of the cerebrospinal fluid is about 7.28 to about 7.32, for intrathecal delivery, a pH within this range may be desired. However, other pHs within the broadest ranges and these subranges may be selected for other route of delivery.

A suitable surfactant, or combination of surfactants, may be selected from among non ionic surfactants that are nontoxic. In one example, a difunctional block copolymer surfactant terminating in primary hydroxyl groups is selected, e.g., such as Pluronic^{®} F68 [BASF], also known as Poloxamer 188, which has a neutral pH, has an average molecular weight of 8400. Other surfactants and other Poloxamers may be selected, i.e., nonionic triblock copolymers composed of a central hydrophobic chain of polyoxypropylene (polypropylene oxide)) flanked by two hydrophilic chains of polyoxyethylene (poly(ethylene oxide)), SOLUTOL HS 15 (Macrogol-15 Hydroxystearate), LABRASOL (Polyoxy capryllic glyceride), polyoxy 10 oleyl ether, TWEEN (polyoxyethylene sorbitan fatty acid esters), ethanol and polyethylene glycol. In one example, the formulation contains a poloxamer.

These copolymers are commonly named with the letter "P" (for poloxamer) followed by three digits: the first two digits x 100 give the approximate molecular mass of the polyoxypropylene core, and the last digit x 10 gives the percentage polyoxyethylene content. Poloxamer 188 may be selected. The surfactant may be present in an amount up to about 0.0005 % to about 0.001% of the suspension.

In one example, the formulation may contain, e.g., buffered saline solution comprising one or more of sodium chloride, sodium bicarbonate, dextrose, magnesium sulfate (e.g., magnesium sulfate -7H20), potassium chloride, calcium chloride (e.g., calcium chloride - 2H20), dibasic sodium phosphate, and mixtures thereof, in water. Suitably, for intrathecal delivery, the osmolarity is within a range compatible with cerebrospinal fluid (e.g., about 275 to about 290); see, e.g., emedicine.medscape.com/-article/2093316-overview. Optionally, for intrathecal delivery, a commercially available diluent may be used as a suspending agent, or in combination with another suspending agent and other optional excipients. See, e.g., Elliotts B^{®} solution [Lukare Medical] The pH of Elliotts B Solution is 6 to 7.5, and the osmolarity is 288 mOsmol per liter (calculated). The composition containing the rAAVhu68.SMN1 gene may be delivered at a pH in the range of 6.8 to 8, or 7.2 to 7.8, or 7.5 to 8. For intrathecal delivery, a pH above 7.5 may be desired, e.g., 7.5 to 8, or 7.8.

The formulation may contain a buffered saline aqueous solution not comprising sodium bicarbonate. Such a formulation may contain a buffered saline aqueous solution comprising one or more of sodium phosphate, sodium chloride, potassium chloride, calcium chloride, magnesium chloride and mixtures thereof, in water, such as a Harvard's buffer. The aqueous solution may further contain Kolliphor^{®} PI 88, a poloxamer which is commercially available from BASF which was formerly sold under the trade name Lutrol^{®} F68. The aqueous solution may have a pH of 7.2.

Alternatively, the formulation may contain a buffered saline aqueous solution comprising 1 mM Sodium Phosphate (Na3P04), 150 mM sodium chloride (NaCl), 3mM potassium chloride (KC1), 1.4 mM calcium chloride (CaC12), 0.8 mM magnesium chloride (MgC12), and 0.001% Kolliphor^{®} 188. See, e.g., harvardapparatus.com/harvard-apparatus-perfusion-fluid.html. Harvard's buffer may be preferred due to better pH stability observed with Harvard's buffer.

The device provided herein avoids the need for one or more permeation enhancers. Examples of suitable permeation enhancers may include, e.g., mannitol, sodium glycocholate, sodium taurocholate, sodium deoxycholate, sodium salicylate, sodium caprylate, sodium caprate, sodium lauryl sulfate, polyoxyethylene-9-laurel ether, or EDTA. A composition may include a carrier, diluent, excipient and/or adjuvant. Suitable carriers may be readily selected by one of skill in the art in view of the indication for which the transfer virus is directed. For example, one suitable carrier includes saline, which may be formulated with a variety of buffering solutions (e.g., phosphate buffered saline). Other exemplary carriers include sterile saline, lactose, sucrose, calcium phosphate, gelatin, dextran, agar, pectin, peanut oil, sesame oil, and water. The buffer/carrier should include a component that prevents the rAAV, from sticking to the infusion tubing but does not interfere with the rAAV binding activity in vivo.

Suitable exemplary preservatives include chlorobutanol, potassium sorbate, sorbic acid, sulfur dioxide, propyl gallate, the parabens, ethyl vanillin, glycerin, phenol, and parachlorophenol. Suitable chemical stabilizers include gelatin and albumin.

In certain examples which are outside the scope of the present invention, a suitable volume (e.g., lcc - 5 cc) of cerebrospinal fluid (CSF) is withdrawn prior to delivery of a selected suspension or solution to a patient. The volume of suspension or solution withdrawn from the CSF may correspond to the volume being delivered to the patient via the device. This may be done following lumbar puncture and/or following insertion of the device. Intravenous (IV) contrast may be administered prior to or during insertion of the device. The patient may be anesthetized, intubated, and positioned on the procedure table.

Suitable volumes for delivery of these doses and concentrations may be determined by one of skill in the art. For example, volumes of about 1 pL to 150 mL may be selected, with the higher volumes being selected for adults. Typically, for newborn infants a suitable volume is about 0.5 mL to about 10 mL, for older infants, about 0.5 mL to about 15 mL may be selected. For toddlers, a volume of about 0.5 mL to about 20 mL may be selected. For children, volumes of up to about 30 mL may be selected. For pre-teens and teens, volumes up to about 50 mL may be selected. In still other examples which are outside the scope of the present invention, a patient may receive an intrathecal administration in a volume of about 5 mL to about 15 mL are selected, or about 7.5 mL to about 10 mL. Other suitable volumes and dosages may be determined. The dosage will be adjusted to balance the therapeutic benefit against any side effects and such dosages may vary depending upon the therapeutic application for which the recombinant vector is employed.

A gene therapy vector may be an AAV-based vector having a dose of about 1 x 109 GC/g brain mass to about 1 x 1012 GC/g brain mass. The dose may be in the range of about 3 x 1010 GC/g brain mass to about 3 x 1011 GC/g brain mass. The dose may be in the range of about 5 x 1010 GC/g brain mass to about 1.85 x 1011 GC/g brain mass. The vector may be delivered in doses of from at least about least 1x109 GCs to about 1 x 1015, or about 1 x 1011 to 5 x 1013 GC. Still other suitable doses of gene therapy vectors or non-vector delivery systems may be readily selected by one of skill in the art.

Similarly, other compositions may be delivered via the device for treatment of various central nervous system injuries, diseases, conditions or disorders.

While the invention has been described with reference to particular embodiments, it will be appreciated that modifications can be made without departing from the scope of the appended claims.

## Claims

1. A minimally-invasive access and delivery medical device for accessing the subarachnoid space of a patient, comprising:
an elongate, flexible microcatheter (10) having proximal and distal ends (16, 12); a guidewire (14) extending through the microcatheter (10) and being slidable relative to the microcatheter (10) such that the guidewire (14) is extendable from the distal end (12) of the microcatheter (10) to a position beyond the distal end (12) and retractable within the microcatheter (10) and such that the microcatheter (10) is advanceable along the guidewire (14) when the guidewire (14) is extended beyond the distal end (12) of the microcatheter (10);
a fiber-optic probe (22) extending through the microcatheter (10) from the distal end (12) to the proximal end (16), the fiber-optic probe (22) having a tip extending adjacent the distal end (12) of the microcatheter (10) and being adjustable relative to the distal end (12) of the microcatheter (10) such that the tip provides an end viewing face (24) that is adjustable in direction toward the guidewire (14); and
a camera (36) connected to the fiber-optic probe (22) adjacent the proximal end (16) of the microcatheter (10) thereby enabling real-time visualization of contents of the subarachnoid space of the patient and guidewire (14) during navigation of the distal end (12) of the microcatheter (10) via the guidewire (14).

2. The minimally-invasive access and delivery medical device according to claim 1, further comprising a steering wire (18, 20) extending through the microcatheter (10) from the proximal end (16) to the distal end (12), the steering wire (18, 20) being fixed to the distal end (12) to enable steering of the distal end (12).

3. The minimally-invasive access and delivery medical device according to claim 2, further comprising a handle (32, 34) connected to the proximal end (16) of the microcatheter (10), said handle (32, 34) providing a housing for the camera (36) and a control lever (40) interconnected to the steering wire (18, 20) for controlling steering of the distal end (12) of the microcatheter (10).

4. The minimally-invasive access and delivery medical device according to claim 1, further comprising first and second steering wires (18, 20) extending through the microcatheter (10) from the proximal end (16) to the distal end (12) and a handle (32, 34) connected to the proximal end (16) of the microcatheter (10), said handle (32, 34) having a control lever (40) interconnected to the first and second steering wires (18, 20) for controlling steering of the distal end (12) of the microcatheter (10).

5. The minimally-invasive access and delivery medical device according to claim 4, wherein the first steering wire (18) connects to or is integral with the second steering wire (20) within the handle (32, 34).

6. The minimally-invasive access and delivery medical device according to claim 1, further comprising a fiber-optic light element extending through the microcatheter (10) from the proximal end (16) to the distal end (12) for projecting light from the distal end (12).

7. The minimally-invasive access and delivery medical device according to claim 1, wherein said microcatheter (10) includes a path for delivery of at least one of a therapy, a gene therapy, a drug, a probe, a medical tool, a lasso, an electrode, and laser ablation element to the distal end (12) of the microcatheter (10).

8. The minimally-invasive access and delivery medical device according to claim 1, wherein said distal end (12) of said microcatheter (10) is in the form of multi-lumen tubing (30) defining separate lumens for at least each of said guidewire (14) and said fiber-optic probe (22).

9. The minimally-invasive access and delivery medical device according to claim 8, wherein the multi-lumen tubing (30) interconnects to intermediate tubing (26) extending to the proximal end (16) of the microcatheter (10), the multi-lumen tubing (30) forming the distal end (12) of the microcatheter (10) being more or less flexible than the intermediate tubing (26).

10. The minimally-invasive access and delivery medical device according to claim 1, wherein the fiber optic probe is encapsulated in a sheath of a thermoplastic polymer.

11. The minimally-invasive access and delivery medical device according to claim 1, wherein the microcatheter (10) is of a size of less than 5 French (Fr) (an outer diameter of less than 1.66 mm (0.07 inch)).

12. The minimally-invasive access and delivery medical device according to claim 1, wherein the microcatheter (10) is of a size of 3.5 French (Fr) or less (an outer diameter of 1.167 mm (0.046 inch) or less).

13. The minimally-invasive access and delivery medical device according to claim 1, wherein the microcatheter (10) is of a size of 3 French (Fr) or less (an outer diameter of 1 mm (0.039 inch) or less).

14. The minimally-invasive access and delivery medical device according to claim 1, wherein the tip of the fiber optic probe (22) is beveled such that the end viewing face (24) is angled relative to a plane extending perpendicular through a longitudinal axis of the microcatheter (10).

15. The minimally-invasive access and delivery medical device according to claim 14, wherein the end viewing face (24) of the fiber optic probe (22) extends at an angle within a range of 12° to 20° relative to a plane extending perpendicular through a longitudinal axis of the microcatheter (10).

## Patentansprüche

1. Minimalinvasive medizinische Zugangs- und Verabreichungsvorrichtung für den Zugang zum Subarachnoidalraum eines Patienten, umfassend:
einen länglichen, flexiblen Mikrokatheter (10) mit einem proximalen und einem distalen Ende (16, 12);
einen Führungsdraht (14), der sich durch den Mikrokatheter (10) erstreckt und relativ zum Mikrokatheter (10) verschiebbar ist, so dass der Führungsdraht (14) aus dem distalen Ende (12) des Mikrokatheters (10) bis zu einer Position jenseits des distalen Endes (12) ausfahrbar und in den Mikrokatheter (10) einziehbar ist, und so dass der Mikrokatheter (10) entlang des Führungsdrahtes (14) vorgeschoben werden kann, wenn der Führungsdraht (14) über das distale Ende (12) des Mikrokatheters (10) hinaus ausgefahren ist;
eine faseroptische Sonde (22), die sich durch den Mikrokatheter (10) vom distalen Ende (12) zum proximalen Ende (16) erstreckt, wobei die faseroptische Sonde (22) eine Spitze aufweist, die sich benachbart zum distalen Ende (12) des Mikrokatheters (10) erstreckt und relativ zum distalen Ende (12) des Mikrokatheters (10) so einstellbar ist, dass die Spitze eine in Richtung zum Führungsdraht (14) einstellbare Endbetrachtungsfläche (24) bereitstellt; und
eine Kamera (36), die mit der faseroptischen Sonde (22) benachbart zum proximalen Ende (16) des Mikrokatheters (10) verbunden ist, wodurch eine Echtzeit-Visualisierung des Inhalts des Subarachnoidalraums des Patienten und des Führungsdrahts (14) während der Navigation des distalen Endes (12) des Mikrokatheters (10) mittels des Führungsdrahts (14) ermöglicht wird.

2. Minimalinvasive medizinische Zugangs- und Verabreichungsvorrichtung nach Anspruch 1, weiter umfassend einen Steuerdraht (18, 20), der sich durch den Mikrokatheter (10) vom proximalen Ende (16) zum distalen Ende (12) erstreckt, wobei der Steuerdraht (18, 20) am distalen Ende (12) befestigt ist, um das Lenken des distalen Endes (12) zu ermöglichen.

3. Minimalinvasive medizinische Zugangs- und Verabreichungsvorrichtung nach Anspruch 2, weiter umfassend einen mit dem proximalen Ende (16) des Mikrokatheters (10) verbundenen Griff (32, 34), wobei der Griff (32, 34) ein Gehäuse für die Kamera (36) und einen Steuerhebel (40) bereitstellt, der mit dem Steuerdraht (18, 20) verbunden ist, um das Lenken des distalen Endes (12) des Mikrokatheters (10) zu steuern.

4. Minimalinvasive medizinische Zugangs- und Verabreichungsvorrichtung nach Anspruch 1, weiter umfassend einen ersten und zweiten Steuerdraht (18, 20), die sich durch den Mikrokatheter (10) vom proximalen Ende (16) zum distalen Ende (12) erstrecken, und einen mit dem proximalen Ende (16) des Mikrokatheters (10) verbundenen Griff (32, 34), wobei der Griff (32, 34) einen Steuerhebel (40) aufweist, der mit dem ersten und zweiten Steuerdraht (18, 20) verbunden ist, um das Lenken des distalen Endes (12) des Mikrokatheters (10) zu steuern.

5. Minimalinvasive medizinische Zugangs- und Verabreichungsvorrichtung nach Anspruch 4, wobei der erste Steuerdraht (18) mit dem zweiten Steuerdraht (20) innerhalb des Griffs (32, 34) verbunden ist oder mit diesem zweiten Steuerdraht einstückig ausgebildet ist.

6. Minimalinvasive medizinische Zugangs- und Verabreichungsvorrichtung nach Anspruch 1, weiter umfassend ein faseroptisches Lichtelement, das sich durch den Mikrokatheter (10) vom proximalen Ende (16) zum distalen Ende (12) erstreckt, um Licht aus dem distalen Ende (12) auszustrahlen.

7. Minimalinvasive medizinische Zugangs- und Verabreichungsvorrichtung nach Anspruch 1, wobei der Mikrokatheter (10) einen Weg zur Verabreichung von mindestens einer Therapie, einer Gentherapie, einem Medikament, einer Sonde, einem medizinischen Instrument, einem Lasso, einer Elektrode und einem Laserablationselement an das distale Ende (12) des Mikrokatheters (10) umfasst.

8. Minimalinvasive medizinische Zugangs- und Verabreichungsvorrichtung nach Anspruch 1, wobei das distale Ende (12) des Mikrokatheters (10) als ein Mehrlumen-Schlauch (30) ausgebildet ist, der separate Lumen für mindestens jeden der Führungsdraht (14) und der faseroptischen Sonde (22) definiert.

9. Minimalinvasive medizinische Zugangs- und Verabreichungsvorrichtung nach Anspruch 8, wobei der Mehrlumen-Schlauch (30) mit einem Zwischenschlauch (26) verbunden ist, der sich bis zum proximalen Ende (16) des Mikrokatheters (10) erstreckt, wobei der das distale Ende (12) des Mikrokatheters (10) bildende Mehrlumen-Schlauch (30) mehr oder weniger flexibel ist als der Zwischenschlauch (26).

10. Minimalinvasive medizinische Zugangs- und Verabreichungsvorrichtung nach Anspruch 1, wobei die faseroptische Sonde in einer Hülle aus einem thermoplastischen Polymer eingekapselt ist.

11. Minimalinvasive medizinische Zugangs- und Verabreichungsvorrichtung nach Anspruch 1, wobei der Mikrokatheter (10) eine Größe von weniger als 5 French (Fr) (einen Außendurchmesser von weniger als 1,66 mm (0,07 Zoll)) hat.

12. Minimalinvasive medizinische Zugangs- und Verabreichungsvorrichtung nach Anspruch 1, wobei der Mikrokatheter (10) eine Größe von 3,5 French (Fr) oder weniger (einen Außendurchmesser von 1,167 mm (0,046 Zoll) oder weniger) hat.

13. Minimalinvasive medizinische Zugangs- und Verabreichungsvorrichtung nach Anspruch 1, wobei der Mikrokatheter (10) eine Größe von 3 French (Fr) oder weniger (einen Außendurchmesser von 1 mm (0,039 Zoll) oder weniger) hat.

14. Minimalinvasive medizinische Zugangs- und Verabreichungsvorrichtung nach Anspruch 1, wobei die Spitze der faseroptischen Sonde (22) so abgeschrägt ist, dass die Endbetrachtungsfläche (24) relativ zu einer sich senkrecht zu einer Längsachse des Mikrokatheters (10) erstreckenden Ebene abgewinkelt ist.

15. Minimalinvasive medizinische Zugangs- und Verabreichungsvorrichtung nach Anspruch 14, wobei sich die Endbetrachtungsfläche (24) der faseroptischen Sonde (22) in einem Winkel zwischen 12° und 20° relativ zu einer Ebene erstreckt, die senkrecht zur Längsachse des Mikrokatheters verläuft.

## Revendications

1. Dispositif médical d'accès et d'administration mini-invasif pour accéder à l'espace sous-arachnoïdien d'un patient, comprenant :
un micro-cathéter allongé et flexible (10), ayant des extrémités proximale et distale (16, 12);
un fil guide (14) s'étendant à travers le micro-cathéter (10) et pouvant coulisser par rapport au micro-cathéter (10) de telle sorte que le fil guide (14) puisse s'étendre depuis l'extrémité distale (12) du micro-cathéter (10) jusqu'à une position au-delà de l'extrémité distale (12) et
puisse être rétracté à l'intérieur du micro-cathéter (10), et de telle sorte que le micro-cathéter (10) puisse être avancé le long du fil guide (14) lorsque le fil guide (14) est étendu au-delà de l'extrémité distale (12) du micro-cathéter (10);
une sonde à fibre optique (22) s'étendant à travers le micro-cathéter (10) depuis l'extrémité distale (12) jusqu'à l'extrémité proximale (16), la sonde à fibre optique (22) ayant une pointe s'étendant à proximité de l'extrémité distale (12) du micro-cathéter (10) et étant réglable par rapport à l'extrémité distale (12) du micro-cathéter (10) de telle sorte que la pointe fournisse une face d'observation d'extrémité (24) qui est réglable en direction vers le fil guide (14); et une caméra (36) reliée à la sonde à fibre optique (22) à proximité de l'extrémité proximale (16) du micro-cathéter (10), permettant ainsi une visualisation en temps réel du contenu de l'espace sous-arachnoïdien du patient et du fil guide (14) pendant la navigation de l'extrémité distale (12) du micro-cathéter (10) via le fil guide (14).

2. Dispositif médical d'accès et d'administration mini-invasif selon la revendication 1, comprenant en outre un fil de pilotage (18, 20) s'étendant à travers le micro-cathéter (10) depuis l'extrémité proximale (16) jusqu'à l'extrémité distale (12), le fil de pilotage (18, 20) étant fixé à l'extrémité distale (12) pour permettre le pilotage de l'extrémité distale (12).

3. Dispositif médical d'accès et d'administration mini-invasif selon la revendication 2, comprenant en outre une poignée (32, 34) reliée à l'extrémité proximale (16) du micro-cathéter (10), ladite poignée (32, 34) fournissant un boîtier pour la caméra (36) et un levier de commande (40) connecté au fil de pilotage (18, 20) pour commander le pilotage de l'extrémité distale (12) du micro-cathéter (10).

4. Dispositif médical d'accès et d'administration mini-invasif selon la revendication 1, comprenant en outre un premier et un deuxième fils de pilotage (18, 20) s'étendant à travers le micro-cathéter (10) depuis l'extrémité proximale (16) jusqu'à l'extrémité distale (12) et une poignée (32, 34) reliée à l'extrémité proximale (16) du micro-cathéter (10), ladite poignée (32, 34) comportant un levier de commande (40) connecté au premier et au deuxième fils de pilotage (18, 20) pour commander le pilotage de l'extrémité distale (12) du micro-cathéter (10).

5. Dispositif médical d'accès et d'administration mini-invasif selon la revendication 4, dans lequel le premier fil de pilotage (18) est relié au deuxième fil de pilotage (20) ou est intégral avec ce deuxième fil de pilotage à l'intérieur de la poignée (32, 34).

6. Dispositif médical d'accès et d'administration mini-invasif selon la revendication 1, comprenant en outre un élément lumineux à fibre optique s'étendant à travers le micro-cathéter (10) depuis l'extrémité proximale (16) jusqu'à l'extrémité distale (12) pour projeter de la lumière depuis l'extrémité distale (12).

7. Dispositif médical d'accès et d'administration mini-invasif selon la revendication 1, dans lequel ledit micro-cathéter (10) comprend un chemin pour l'administration à l'extrémité distale (12) du micro-cathéter (10) d'au moins un élément parmi un traitement, une thérapie génique, un médicament, une sonde, un outil médical, un lasso, une électrode et un élément d'ablation laser.

8. Dispositif médical d'accès et d'administration mini-invasif selon la revendication 1, dans lequel ladite extrémité distale (12) dudit micro-cathéter (10) se présente sous la forme d'un tube à lumières multiples (30) définissant des lumières séparées pour au moins chacun dudit fil guide (14) et de ladite sonde à fibre optique (22).

9. Dispositif médical d'accès et d'administration mini-invasif selon la revendication 8, dans lequel le tube à lumières multiples (30) est relié à un tube intermédiaire (26) s'étendant jusqu'à l'extrémité proximale (16) du micro-cathéter (10), le tube multi-lumière (30) qui forme l'extrémité distale (12) du micro-cathéter (10) étant plus ou moins flexible que le tube intermédiaire (26).

10. Dispositif médical d'accès et d'administration mini-invasif selon la revendication 1, dans lequel la sonde à fibre optique est encapsulée dans une gaine en polymère thermoplastique.

11. Dispositif médical d'accès et d'administration mini-invasif selon la revendication 1, dans lequel le micro-cathéter (10) a une taille inférieure à 5 French (Fr) (un diamètre extérieur inférieur à 1,66 mm (0,07 pouce)).

12. Dispositif médical d'accès et d'administration mini-invasif selon la revendication 1, dans lequel le micro-cathéter (10) a une taille égale ou inférieure à 3,5 French (Fr) (un diamètre extérieur égal ou inférieur à 1,167 mm (0,07 pouce)).

13. Dispositif médical d'accès et d'administration mini-invasif selon la revendication 1, dans lequel le micro-cathéter (10) a une taille égale ou inférieure à 3 French (Fr) (un diamètre extérieur égal ou inférieur à 1 mm (0,039 pouce)).

14. Dispositif médical d'accès et d'administration mini-invasif selon la revendication 1, dans lequel l'extrémité de la sonde à fibre optique (22) est biseautée de telle sorte que la face d'observation d'extrémité (24) forme un angle par rapport à un plan s'étendant perpendiculairement à l'axe longitudinal du micro-cathéter (10).

15. Dispositif médical d'accès et d'administration mini-invasif selon la revendication 14, dans lequel la face d'observation d'extrémité (24) de la sonde à fibre optique (22) s'étend selon un angle compris entre 12° et 20° par rapport à un plan s'étendant perpendiculairement à l'axe longitudinal du micro-cathéter (10).
